# EUROPEAN PATENT SPECIFICATION

(11) **EP 2 484 341 B1**
(45) Date of publication and mention of the grant of the patent: **22.10.2014**
(21) Application number: 10818722.0
(22) Date of filing: 14.09.2010
(51) Int. Cl.: A61K 8/49, A61K 31/381, A61Q 19/02, C07D 333/46

(54) **MELANIN PRODUCTION INHIBITOR**
MELANINPRODUKTIONSHEMMER
INHIBITEUR DE LA PRODUCTION DE MÉLANINE

(30) Priority: 28.09.2009 JP 2009223466
(43) Date of publication of application: 08.08.2012
(73) Proprietor: FUJIFILM Corporation, Tokyo 106-0031 (JP)
(72) Inventor: HONMA Toshiyuki, Kanagawa (JP); KUBO Toshiaki, Kanagawa (JP)
(74) Representative: Morpeth, Fraser Forrest
(86) International application number: PCT/JP2010/065859
(87) International publication number: WO 2011/037047

(56) References cited:
- WO-A1-2009/002977
- JP-A- 2006 188 463
- JP-A- 2007 527 850
- JP-A- 2008 120 774

## Description

### Technical Field

The present invention relates to a melanin production inhibitor which inhibits skin melanism induced by ultraviolet rays or the like, and a whitening cosmetic exhibiting an excellent whitening effect and a high safety. Furthermore, the invention relates to use of salacinol as a melanin production inhibitor in a whitening cosmetic exhibiting an excellent whitening effect and a high safety which inhibits skin melanism induced by ultraviolet rays.

### Background Art

Recently, prevention or recovery of skin color change such as sunburn, freckles, and ephelides, i.e., so-called whitening has been attracted attention.

When skin is exposed to ultraviolet rays included in sun light, ultraviolet lamps, and the like, the skin gets inflamed and skin tissues are injured to lose so-called shiny complexion, fine texture, wetness, and the like. Particularly, when dermis is injured by ultraviolet rays, wrinkles and sag are generated and so-called photoaging may be caused.

Active oxygen generated by ultraviolet exposure and various factors released from skin cells under the influence thereof enhance tyrosinase activity in melanocyte. Melanin that participates color tone of the skin is produced through oxidation of tyrosine by the action of tyrosinase in melanocyte. When tyrosinase is activated by ultraviolet rays, it is considered that melanin is excessively produced and transferred to epidermal cells and thus the color tone of the skin is changed and blackened. Accordingly, in order to obtain a whitening effect, it is required to inhibit melanin production.

Hitherto, there have been proposed whitening cosmetics containing ascorbic acid, kojic acid, arbutin, ellagic acid, or a derivative thereof, or various plant extracts.

On the other hand, salacinol represented by the structural formula (1) to be mentioned later is a compound discovered from plants belonging to the genus *Salacia.* It has been handed down by tradition that roots and trunks of plants belonging to the genus *Salacia* are effective for prevention and initial treatment of diabetes in traditional medicine ayurveda in India and Sri Lanka. In recent years, it has been reported that plants belonging to the genus *Salacia* have an inhibitory action on elevation of a blood glucose level and the action mechanism is attributable to a sugar absorption inhibitory action based on α-glucosidase activity inhibition.

Moreover, application of crude extracts of plants belonging to the genus *Salacia* to so-called whitening agents has also been proposed (Patent Documents 1 and 2).

### Background Art Documents

### Patent Documents

Patent Document 1: JP-A-2006-188463
Patent Document 2: JP-A-2008-120774

### Disclosure of the Invention

### Problems that the Invention is to Solve

The ingredients hitherto known to have whitening action are poor in stability and solubility in formulation systems and also are not sufficient in activity in the living body. Furthermore, they cause eruption on the skin, for example, and thus are not satisfactory also in view of safety. Even when a crude extract of plants belonging to the genus *Salacia* is used, whitening action is not sufficient and also, when it is used in a high concentration in order to enhance the action, cytotoxicity comes about, so that it has been revealed to be unsuitable.

Accordingly, a whitening ingredient exhibiting a high whitening action and a low cytotoxicity.

### Means for Solving the Problems

In consideration of such circumstances, as a result of the extensive studies, the present inventors have found that salacinol represented by the formula (1) has an excellent melanin production inhibitory action and also has high stability and safety. Thereby, it becomes possible to provide a safe and stable whitening cosmetic.

Namely, the present invention has the following constitution.
1. A compound represented by the following structural formula (1) for use as a melanin production inhibitor. 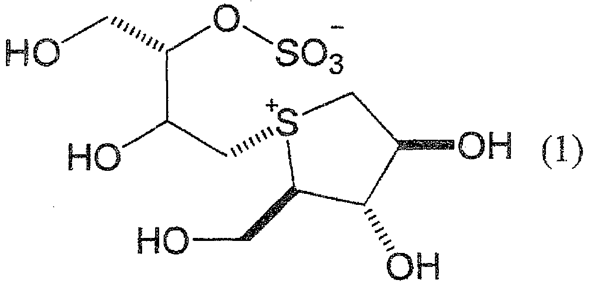
2. A whitening cosmetic comprising the compound of structural formula (1) according to 1 above.
3. The compound described in 1. above for use as an active ingredient for melanin production inhibition in the production of a whitening cosmetic.

### Advantage of the Invention

According to the invention, there is provided a melanin production inhibitor exhibiting a high melanin production inhibitory effect and having a high stability and safety, specifically, a whitening cosmetic inhibiting skin color change induced by ultraviolet rays or the like.

### Brief Description of the Drawings

[FIG. 1]

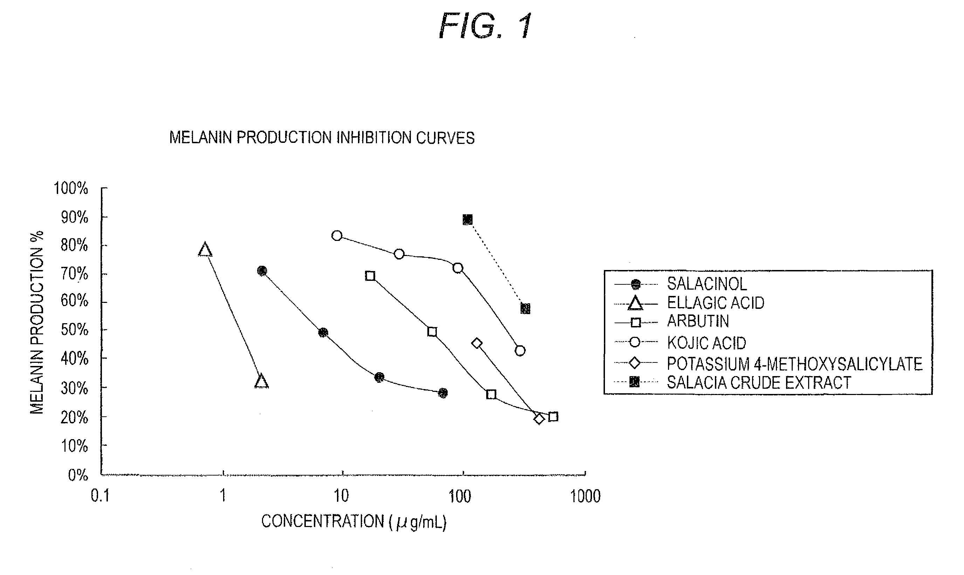

   FIG. 1 is a drawing showing melanin production inhibitory curves of salacinol and comparative controls.
[FIG. 2]

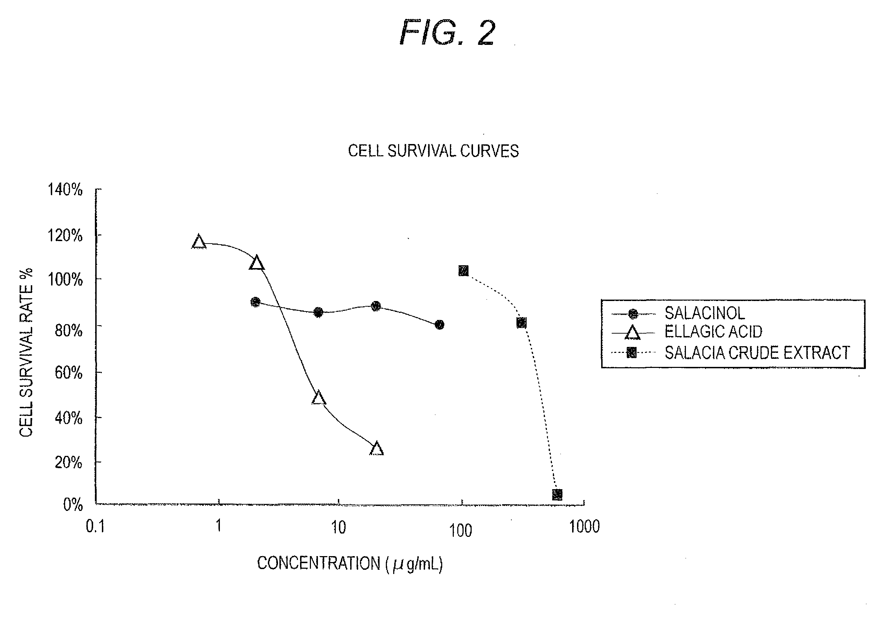

   FIG. 2 is a drawing showing cell survival curves of salacinol and comparative controls.

### Mode for Carrying Out the Invention

The following will explain embodiments of the present invention in detail.

In order to explain the invention in detail, production examples and experimental examples of compounds to be used in the invention are mentioned as Examples but the invention is not limited thereto.

The melanin production inhibitor of the invention contains a compound represented by the following structural formula (1) as an active ingredient.

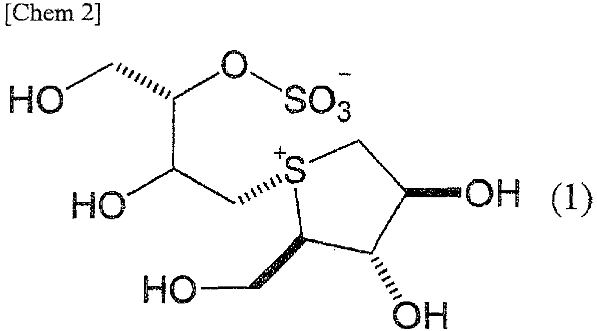

The compound represented by the structural formula (1) is called salacinol and can be obtained by isolation and purification from plants belonging to the genus *Salacia* and extracts thereof using a known method, for example preparative chromatography.

The plants belonging to the genus *Salacia* are plants belonging to *Celastraceae* which grow wild mainly in Sri Lanka, India, and Southeast Asia area. More specifically, one or more kinds of plants selected from *Salacia reticulate, Salacia oblonga, Salacia prinoides, Salacia chinensis, Salacia latifolia, Salacia burunoniana, Salacia grandiflora,* and *Salacia macrosperma* are used.

The extract of the plants belonging to the genus *Salacia* means a ground product, a dried product, an extract, a dried powder thereof (extract powder), or the like of edible parts such as roots, trunks, leaves, flowers, and fruits. It is also suitable to mix and use one or more kinds of the parts. More preferably, an extract powder extracted from roots and trunks is used.

The extract powder is a dried product of one obtained by solvent extraction from the aforementioned edible parts and the like. The extraction solvent may be selected from the group consisting of water or alcohols such as methanol and ethanol, or mixed solvent of water and alcohols or ketones such as acetone. Preferably, water, an alcohol, or an aqueous alcohol is used. More preferably, as the extraction solvent, hot water or ethanol or an aqueous ethanol is used. Regarding the alcohol concentration of the above aqueous alcohol, one having a concentration of 30 to 90% by mass, preferably 40 to 70% by mass may be used.

As the drying method, spray drying, freeze-drying, and the like may be mentioned without limitation thereto.

Moreover, those synthesized using known synthetic methods, for example, the method described in JP-A-2009-528299 (WO 2007-098567) and the like can be also used.

The blending amount of salacinol in the invention is preferably 0.0005 to 10.0% by mass (hereinafter simply described as %) on the basis of the total amount of the melanin production inhibitor or the whitening cosmetic. The mixing amount in this range is preferred since the objective effect of the invention becomes sufficient.

Moreover, in the invention, at the production of the whitening cosmetic, in the case where salacinol is used as an active ingredient for melanin production inhibition, the blending amount is preferably 0.0005 to 10.0% by mass on the basis of the total amount of the whitening cosmetic similarly to the above. Salacinol to be blended may be any form such as a solid form or a solution form and may be added and blended at an appropriate timing during the production process of the whitening cosmetic.

Whitening is more precisely an action to prevent or recover skin melanism, specifically change in color tone/pigment of the skin such as sunburn, freckles, and ephelides induced by excessive production of melanin and to maintain or restore the color tone of the skin, for example, to whiten the skin.

By using the melanin production inhibitor of the invention or the whitening cosmetic containing the same to the skin, a whitening effect can be obtained and also pigment macules (e.g., senile pigment macules (freckles), ephelides (freckles), chloasma, pigmentation after inflammation, nevus, etc.) can be treated.

The whitening cosmetic of the invention may be used as a cosmetic in the form of generally applying to the skin and also as a bath additive.

In the both cases of the cosmetic and the bath additive, the dosage form is not particularly limited and they can be used in commonly used form. For example, aqueous solutions, emulsions (W/O type or O/W type), or powders such as granules, tablets, or the like are considered and excipients and the like may be further incorporated. More specifically, creams, milky lotions, skin lotions, face packs, gels, sticks, sheets, poultices, and the like may be mentioned.

Any of them can be produced in usual manners and, for example, in the case of milky lotions and the like, they can be produced by heating and dissolving an aqueous phase and an oily phase separately and dispersing and emulsifying them, followed by cooling.

Moreover, in the whitening cosmetic of the invention, hydroquinone, arbutin, ellagic acid, a vitamin C derivative, a biphenyl derivative, 4-(4-hydroxyphenyl)-2-butanol, and the like which have been already known and which are other whitening agent, may be blended with appropriate combination to enhance or reinforce the effect.

In this regard, in the cosmetic of the invention, more precisely, in the cosmetic to be used as a skin external preparation, in addition to the above, other ingredients usually containable in cosmetics may be incorporated. For example, there may be incorporated coloring matters (e.g., tar-based coloring matters), pigments (e.g., iron oxides, titanium oxides, etc.), humectants (e.g., hyaluronic acid, glycerin, polyhydric alcohols, sugar alcohols, etc.), antiseptics (paraben etc.), anionic surfactants (polyoxyethylene alkyl ether, polyoxyethylene fatty acid esters, fatty acid soaps, sodium cetyl sulfate, etc.), nonionic surfactants (polyoxyethylene polyhydric alcohol fatty acid esters, polyoxyethylene hardened caster oil, polyhydric alcohol fatty acid esters, polyglycerin fatty acid esters, etc.), cationic surfactants (tetraalkylammonium salts etc.), amphoteric surfactants (betaine type, sulfobetaine type, sulfoamino acid type, sodium N-stearoyl-L-glutamate, etc.), natural substance-based surfactants (lecithin, lysophosphatidylcholine, etc.), natural polymers (gelatin, casein, starch, gum arabic, karaya gum, guar gum, locust bean gum, tragacanth gum, quince seed, pectin, carrageenan, sodium alginate, etc.), semisynthetic polymers (methyl cellulose, hydroxyethyl cellulose, hydroxypropyl cellulose, sodium carboxymethylcellulose, ethyl cellulose, etc.), synthetic polymers (polyvinyl alcohol, polyvinyl methyl ether and copolymers, polyvinylpyrrolidone, poly(sodium acrylate), carboxyvinyl polymers, polyethylene oxide polymers, etc.), thickeners (xanthen gum etc.), antioxidants (dibutylhydroxytoluene etc.), and the like.

### Examples

The following will explain the invention based on Examples and Production Examples in detail. The invention is not limited thereto.

### Production Example 1 [Isolation and Purification of Salacinol]

One kilogram of an extract of plants belonging to the genus *Salacia* was distributed into ethyl acetate/methanol and a methanol fraction was concentrated under reduced pressure. An ingredient was separated by preparative chromatography on an amide column to obtain 500 mg of salacinol. Obtained salacinol was measured on NMR, MS, and the like and identified by comparison with literature data. The following show conditions for the preparative chromatography.
Column: TOSOH TSK-GEL Amide 80 10µl φ21.5×30 cm
Flow rate: 15 mL/min
Mobile phase: 90% acetonitrile (v/v) (0.1% acetic acid)
Detection: Negative ESI 333

Using salacinol obtained in Production Example 1, the following melanin production inhibition test and cell toxicity test were performed. As comparative controls, a *Salacia* crude extract and known whitening active ingredients were used.

### Example 1

### [Melanin Production Inhibition Test]

B16 melanoma cells were seeded on 10% (v/v) FBS-containing MEM medium (minimum basal medium containing fetal bovine serum) so as to be 2.5×10⁵ cells/well in a 12-well culture plate and were cultivated for 24 hours in a usual manner.

After the pre-cultivation, the medium was replaced with a test medium and cultivation was carried out for 72 hours. As the test medium, there was employed a medium obtained by adding 0.25 mmol/L of theophylline, 0.5% (v/v) of dimethyl sulfoxide to the medium for the pre-cultivation, and each of various evaluation samples so as to be a concentration described in Table.

The *Salacia* crude extract was prepared by the following method. After 0.5 kg of root and trunk parts of *Salacia reticulate, Salacia oblonga,* and *Salacia chinensis* were pulverized, 10 L of water was added thereto and extraction was performed under conditions of 100°C and 1 hour. After the obtained extract solution was filtrated through a 100-mesh filter, spray drying was performed and a product obtained as 50 g of an extract powder was used.

As ellagic acid, ellagic acid dihydrate manufactured by Wako Pure Chemical Industries, Ltd. was used.

As arbutin, β-arbutin manufactured by Sigma-Aldrich was used.

After the cultivation was finished, the cells were washed with PBS (phosphate buffer) and then a 1 mol/L aqueous sodium hydroxide solution containing 10% (v/v) dimethyl sulfoxide was added thereto. After the whole was subjected to an ultrasonic treatment at 50°C for 30 minutes, absorbance of the solution at a wavelength of 400 nm was measured and was taken as a melanin amount in a well. Moreover, at the same time, measurement of the number of living cells by MTT assay was carried out in another culture plate (test conditions are in accordance to Example 2). Finally, the melanin amount in the well was normalized by the measurement value by the MTT assay and the resulting amount was taken as a melanin amount per unit cell.

A melanin production inhibition rate (%) in the case where the evaluation sample was added was determined, the melanin amount in the case where no evaluation sample was added being regarded as 100.

### [Results]

The results are shown in Table 1. Also, melanin production inhibition curves of salacinol and the comparative controls are shown in FIG. 1. From the melanin production inhibition curves, IC50 values at which 50% of the melanin production was inhibited were read out. The results are shown in Table 2.

**Table 1 Melanin Production Inhibition Test**

| Salacinol (µg/mL) | Melanin Production | | Ellagic acid (µg/mL) | Melanin Production | | Arbutin (µg/mL) | Melanin Production |
|---|---|---|---|---|---|---|---|
| 0 | 100.0% | | 0 | 100.0% | | 0 | 100.0% |
| 1.998 | 71% | | 0.67646 | 79% | | 16.338 | 69.5% |
| 6.66 | 50% | | 2.02938 | 33% | | 54.46 | 49.9% |
| 19.98 | 34% | | | | | 163.38 | 28.4% |
| 66.6 | 29% | | | | | 544.6 | 20.7% |
| | | | | | | | |

| Kojic acid (µg/mL) | Melanin Production | | Potassium 4-methoxysalicylate (µg/mL) | Melanin Production | | *Salacia* crude Extract (µg/mL) | Melanin Production |
|---|---|---|---|---|---|---|---|
| 0 | 100.0% | | 0 | 100.0% | | 0 | 100.0% |
| 8.5266 | 83.6% | | 123.69 | 46.0% | | 100 | 90% |
| 28.422 | 77.2% | | 412.3 | 20.1% | | 300 | 59% |
| 85.266 | 72.8% | | | | | | |
| 284.22 | 43.4% | | | | | | |

**Table 2**

| | µg/mL |
|---|---|
| Salacinol | 6.3 |
| Ellagic acid | 1.5 |
| Arbutin | 54 |
| Kojic acid | 240 |
| Potassium 4-methoxysalicylate | 80 |

Salacinol of the invention showed a concentration-dependent and remarkable melanin production inhibitory effect. Moreover, it was revealed that the effect is remarkably strong even in the comparison with arbutin, kojic acid, and potassium 4-methoxysalicylate that are existing whitening active ingredients.

Although the *Salacia* crude extract also showed a melanin production inhibition effect, cytotoxicity was severe when the concentration exceeds 300 µg/mL as shown in Example 2, so that calculation of IC50 was impossible.

### Example 2

### [Cytotoxicity Test]

B16 melanoma cells were seeded on 10% (v/v) FBS-containing MEM medium so as to be 1.25×10⁵ cells/well in a 24-well culture plate and were cultivated for 24 hours in a usual manner.

After the pre-cultivation, the medium was replaced with a test medium and cultivation was carried out for 72 hours. As the test medium, there was employed a medium obtained by adding 0.25 mmol/L of theophylline, 0.5% (v/v) of dimethyl sulfoxide to the medium for the pre-cultivation, and each of various evaluation samples so as to be a concentration described in Table.

After the cultivation was finished, the cells were washed with PBS and then a 0.5 mg/mL MTT solution was added in an amount of 500 µL/well and the temperature was kept for 2 hours in an incubator. Thereafter, 1.5 mL of dimethyl sulfoxide was added thereto and then absorbance at a wavelength of 570 nm was measured and taken as an index of the number of living cells.

A cell revival rate (%) in the case where the evaluation sample was added was determined, the number of living cells in the case where no evaluation sample was added being regarded as 100.

### [Results]

The results are shown in Table 3. Also, cell revival curves of salacinol and the comparative controls are shown in FIG. 2.

**Table 3 Melanin Production Inhibition Test**

| Salacinol (µg/mL) | Cell survival rate | | Ellagic acid (µg/mL) | Cell survival rate | | *Salacia* crude extract (µg/mL) | Cell survival rate |
|---|---|---|---|---|---|---|---|
| 0 | 100.0% | | 0 | 100.0% | | 0 | 100.0% |
| 1.998 | 89.6% | | 0.67646 | 117.4% | | 100 | 103% |
| 6.66 | 85.0% | | 2.02938 | 107.3% | | 300 | 80% |
| 19.98 | 87.9% | | 6.7646 | 48.1% | | 600 | 4% |
| 66.6 | 78.8% | | 20.2938 | 25.8% | | | |

Salacinol of the invention exhibited almost no cytotoxicity even in the concentration region (up to 66.6 µg/mL) at which up to 29% of the melanin production was inhibited. On the other hand, ellagic acid that shows a melanin production inhibition effect stronger than that of salacinol exhibited cytotoxicity in a high concentration region (up to 20.3 µg/mL). The *Salacia* crude extract also exhibited concentration-dependent cytotoxicity.

Moreover, cytotoxicity was observed at a high concentration region also in arbutin, kojic acid, potassium 4-methoxysalicylate which were used as comparative controls in Example 1 (data are not shown).

From these facts, it was revealed that salacinol is a whitening agent exhibiting an excellent melanin production inhibitory effect and a high safety.

### Industrial Applicability

According to the present invention, there is provided a melanin production inhibitor exhibiting an excellent melanin production inhibitory effect and having high stability and safety, specifically, a whitening cosmetic which inhibits skin color change induced by ultraviolet rays or the like.

While the invention has been described in detail and with reference to specific embodiments thereof, it will be apparent to one skilled in the art that various changes and modifications can be made therein without departing from the scope defined by the claims.

## Claims

1. A compound represented by the following structural formula (1) for use as a melanin production inhibitor.

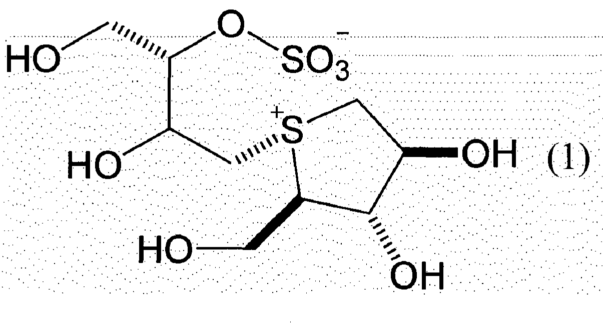

2. A whitening cosmetic comprising the compound of structural formula (1) according to claim 1.

3. A composition, comprising a compound represented by the following structural formula (1) for use in inhibiting melanin production.

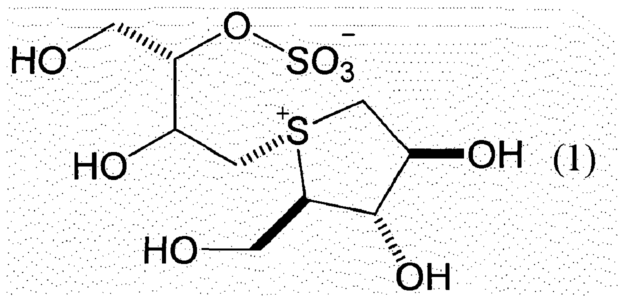

4. The composition according to claim 3, wherein the compound represented by the structural formula (1) is a synthesized compound or isolated and purified compound.

## Patentansprüche

1. Verbindung, wiedergegeben durch die folgende Strukturformel (1), zur Verwendung als Inhibitor der Melaninproduktion.

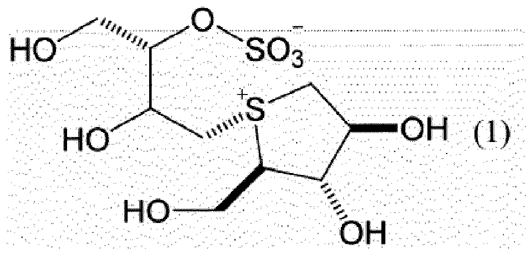

2. Aufhellendes Kosmetikum, welches die Verbindung der Strukturformel (1) nach Anspruch 1 umfasst.

3. Zusammensetzung, umfassend eine durch die folgende Strukturformel (1) wiedergegebene Verbindung, zur Verwendung beim Inhibieren der Melaninproduktion.

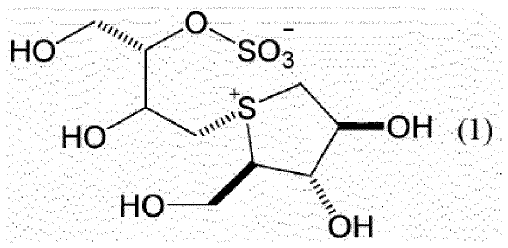

4. Zusammensetzung nach Anspruch 3, wobei es sich bei der durch die Strukturformel (1) wiedergegebenen Verbindung um eine synthetisierte Verbindung oder eine isolierte und aufgereinigte Verbindung handelt.

## Revendications

1. Composé représenté par la formule structurale suivante (1) pour utilisation en tant qu'inhibiteur de production de mélanine.

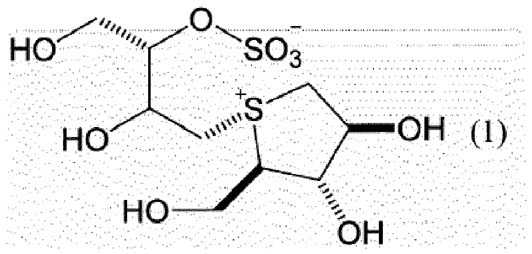

2. Cosmétique de blanchiment comprenant le composé de formule structurale (1) selon la revendication 1.

3. Composition comprenant un composé représenté par la formule structurale suivante (1) pour utilisation dans l'inhibition de la production de mélanine.

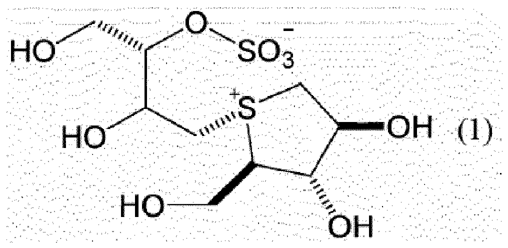

4. Composition selon la revendication 3, dans laquelle le composé représenté par la formule structurale (1) est un composé synthétisé ou un composé isolé et purifié.
